(19) Europäisches Patentamt
European Patent Office
Office européen des brevets

(11) **EP 3 023 792 A1**

(12) **EUROPEAN PATENT APPLICATION**

(43) Date of publication:
**25.05.2016 Bulletin 2016/21**

(51) Int Cl.:
*G01N 33/68* (2006.01)     *G01N 33/543* (2006.01)

(21) Application number: **14306835.1**

(22) Date of filing: **18.11.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **BIOSIMS TECHNOLOGIES**
**76000 Rouen (FR)**

(72) Inventors:
• **Heuclin, Christine**
  **76000 Rouen (FR)**
• **Legent, Guillaume**
  **76116 Martainville Epreville (FR)**

(74) Representative: **Jolly, Christophe et al**
**Ernest Gutmann - Yves Plasseraud SAS**
**3, rue Auber**
**75009 Paris (FR)**

(54) **Spot control**

(57)    The present invention relates to biochips for use in surface mass spectrometry for quantitating a target molecule in a test sample. These biochips are comprised of a substrate to which are attached at least one assay spot(s) containing capture molecules and having at least three control spots containing a signal control molecule having different concentrations of signal control molecules. Kits containing the biochips and methods of using the calibrated biochips are also disclosed, as well as methods of quantitating a target molecule.

**FIGURE 1**

EP 3 023 792 A1

**Description**

**FIELD OF THE INVENTION**

The present invention relates to biochips for use in surface mass spectrometry or similar spectroscopic techniques for quantitating a target molecule in a test sample comprising a substrate to which are attached at least one assay spot(s) containing capture molecules and comprising at least three control spots containing a signal control molecule each control spot having different concentrations of signal control molecules. The present invention also relates to kits containing the biochips and methods of quantitating target molecules using the biochips.

**BACKGROUND OF THE PRESENT INVENTION**

Biochips comprising spots to assay molecules on microarrays have emerged as a promising approach for a wide variety of applications and are increasingly becoming an important tool for the detection and quantification of molecules, molecule-molecule interactions, DNA, RNA, protein-phospholipid interactions, small molecule targets and substrates for protein kinases.

There are four general types of biochips that are currently used in the art; analytical microarrays, functional microarrays, tissue microarrays and reverse phase microarrays. Analytical microarrays are the most common microarray that is used to measure a complex mixture of molecules in order to measure binding affinities, specificities and expression levels. In this technique the microarray has probes such as antibodies, nucleotides, peptides, aptamers or affibodies arranged on a glass slide or chip and the array is probed with a test solution or other laboratory sample. Analytical microarrays are generally used for clinical diagnosis.

Functional microarrays are composed of full length functional proteins or protein domains. They are used to study protein-protein interactions and biochemical activities of an entire proteome.

Reverse Phase microarrays use lysates from various isolated tissues of interest, which are arrayed on a slide and then probed with antibodies, RNA, DNA or other probes against the target of interest. The probes are detected using chemiluminescent, fluorescent, colormetric or isotopic assays. Reverse phase protein microarrays allow for the determination of the presence of altered proteins that may be the result of disease.

Tissue biochips consist of dissected tissues deposited on a slide, wherein the tissue is probed with labeled molecules to detect or quantify the target molecule of interest.

Biochips comprising protein arrays provide a powerful technology which have a high density of molecules and allow a systematic probing of biochemical activities. In principle microarray experiments have opened a door to a vast amount of data at a relatively fast speed and at low costs. However, there still remains a number of key technical issues that have to be resolved in order that the results obtained reach their full potential. For instance, the interpretation of data requires the capability of using image analysis methods to accurately translate image intensity values to true concentration values. Inter- and intra-array technical variability exists, as well as the introduction of artifacts that can distort the signals and affect the correct identification of proteins.

The standardization of biomolecule microarrays poses a problem in the art since most of the methods of the scientific community for preparing and analyzing biochips are not equivalently standardized. Hence variation exists among different laboratories.

Olle et al (2005, Mol And Cell Prot, 4.11 1664-1672) describe a strategy for limiting the variations in the assay using biochips that is called a double system of labeling. Once the hybridization probe or target is placed on the biochip, it is placed in contact with labeled antibodies with a fluorophore that is used to quantify the probe or target and another labeled antibody with a different fluorophore that is used to quantify the targets. This method permits to normalize the number of targets relative to the number of probes, but does not take into account the variations in the emission of the scanner between two biochips.

WO2010/009387 describes a method for normalizing the results obtained with biochips having a mixture of targets at their surface. The method consists of using two probes for each spot, the first probe directed towards the target of interest and labeled with a fluorophore, the second directed towards a known target known to be in constant amount in the mixture of targets (housekeeping proteins) and marked with a different fluorophore. This method permits to control the differences of different deposits between many spots but does not permit to calibrate the scanner.

However, there are problems associated with testing and quantifying different molecular targets using fluorescent assays. The general procedure is to conjugate a fluorophore to a probe and use the fluorescence as an indicator of the presence of the probe in the biological sample. Thus instrumentation to detect the fluorescent probes such as digital fluorescence microscopy and image analysis or LED induced fluorometers has advanced in the art. However one of the main problems with using fluorescent detection is that the fluorescent probe is light sensitive and in many instances produces an unstable signal. Therefore the calibration of the biochip has to be done at the last minute and the calibration of the curve for quantitation has to be performed per biochip for each concentration on the curve.

In the case where one wishes to use the biochips to compare the concentration of targets or probes in many test solutions, it is customary to use a biochip test solution and measure the signal from the labeled targets or probes. The signal measured in the test solution is compared to the all of the other test solutions. A principle bias of this method is that the signal measures for each control solution and/or each test solution cannot be measured simultaneously since each biochip is analyzed individually.

Furthermore, the variations in intensity of the scanner between each measurement leads to an uncertainty in each measurement and the potential high variations in coefficients and hence different quantitative results.

Thus, there is a need in the art to provide a biochip that can control the variables in an assay that uses a biochip to reduce the variation of assays performed in different laboratories.

There is also a need in the art to provide quantitation methods to analyze molecules that are accurate, are stable and do not require use of an unstable label.

Thus it is an object of the present invention to provide biochips that can be used for quantifying molecules using surface mass spectrometers or similar surface spectroscopy such as of nano-Secondary Ion Mass Spectrometer (nano-SIMS), Time of Flight Secondary Ion Mass Spectrometer (TOF-SIMS), Time of Flight Matrix-Assisted Laser Desorption/Ionization (Maldi-Tof), electron spray ionization mass spectrometry (ESIMS), Ion Mobility Spectrometer (IMS), Secondary Ion Mass Spectrometer (SIMS), Matrix-Assisted laser Desorption/Ionization (MALDI), Surface Enhanced Laser Desorption/Ionization (SELDI), Surface-Assisted Laser Desorption/Ionization (SALDI), Auger Electron Spectroscopy (AES), Energy-Dispersive X-ray Spectroscopy (EDS or EDX), X-ray Photoelectron Spectroscopy (XPS) or Dynamic Secondary Ion Mass Spectrometer (D-SIMS).

Another object of the invention is to provide quantitative results of molecules in test samples that are more accurate than using other methods and instrumentation known in the art.

Yet another object of the present invention is to provide biochips with controls that can be easily stored for future use under unspecific conditions, which are stable.

These needs and other objects are achieved by the present invention as evidenced by the summary of the invention, description of the preferred embodiments and the claims.

## SUMMARY OF THE INVENTION

The present invention provides a biochip for use in surface mass spectrometry or similar spectroscopic techniques for quantitating a target molecule in a test sample comprising a substrate to which are attached at least one assay spot(s) containing capture molecules and comprising at least three control spots containing a signal control molecule each control spot having different concentrations of signal control molecules.

In one aspect the capture molecules and signal control molecules are attached to the substrate of the biochip through a functionalized moiety such as through covalent or non covalent bonds or alternatively the capture and signal control molecules are functionalized for attachment to the substrate of the biochip.

The signal control molecule having different concentrations can be labeled with a first signal source. This first signal source can be an element of iodine, bromine, fluorine, sulfur gold, iron, gold, iron, selenium, arsenic, lead, boron, silver, copper, zinc, nickel or lanthanides. The labeled signal control molecule does not bind to the target molecule and is attached to the substrate through a functionalized moiety as described herein.

In yet another aspect the at least three control spots containing a signal control molecule can also contain a dilution molecule. This dilution molecule can be used to dilute the control signal and can be diluted in a similar concentration as the capture molecule on the biochip. This dilution molecule can be unlabeled or labeled with a second signal source. This second signal source is different from the first signal source and is selected from iodine, bromine, fluorine, sulfur gold, iron, gold, iron, selenium, arsenic, lead, boron, silver, copper, zinc, nickel or lanthanides.

The capture molecules are attached to the substrate and bind specifically with the target molecules. The capture molecule(s) and signal control molecules can be antibodies, nucleic acids, aptamers, affibodies, proteins, molecular imprinted polymers, enzymes, ligands, glycans, lectins, lipids, polyamines, phages, viruses, chemicals or combinations thereof. In one aspect the capture molecules and signal control molecules on the biochip are antibodies.

The substrate of the biochip can be fabricated from a material selected from the group of plastics, silicon, fused quartz, soda lime glass, ceramics, pyrex, metals, glassy carbon and polymeric materials. In one aspect the substrate is a silicon wafer. The substrate can be in the shape of a rectangle, a square, a circle, a triangle, an oval, a pentagon, an octoagon or a hexagon. The spots on the biochip can be microfabricated or nanofabricated using various nanoarrayers or microarrayers.

The test sample can be a histological sample such as a tissue or a tumor or an organ or skin. The test sample can also be a lysate of a tissue, or a lysate of a tumor or a lysate of an organ or a lysate of skin. It can also be a cytological sample, such as is a cell, cell lysate or cell culture. In another aspect the test sample can be a biological sample such as blood, serum, plasma, cerebrospinal fluid, seminal fluid, amniotic fluid, bile, gastric juices, saliva, tears, sweat, sputum, semen, peritoneal fluid, pericardial fluid, urine, feces, hair or nail clippings. In yet another aspect the test sample can be an environmental sample such as air, water, food, mining waste, pulp and paper waste or general manufacturing waste.

The test sample contains a target molecule. The target molecule can be selected from the group of a protein, a peptide, a nucleic acid, a carbohydrates, a lipid, a polysaccharide, a glycoprotein, a hormone, an antigen, a pathogen, a toxic substance, a drug, a dye, a nutrient, an enzyme, a rheumatoid factor, a tumor marker, a microorganism, an opiate, a viral epitope, cholesterol, a heavy metal, a vitamin, an allergy, a neurodegenerative disorder, an electrolyte, a glycan, a polyamine, a fatty acid, a sugar, a chemical trace metals and mixtures thereof.

The target molecule can also be selected from the group of emergent chemicals, organic contaminants, mold, fungus, asbestos, nitrogen compounds, pesticides, herbicides, sulfates, bacteria, methylcyclohexane methanol, polychlorinated biphenyls (PCBs) and mixtures thereof.

The at least one assay spot containing the capture molecules binds to the target molecules. The target molecules can be selected from the group of a protein, a peptide, a nucleic acid, a carbohydrates, a lipid, a polysaccharide, a glycoprotein, a hormone, an antigen, a pathogen, a toxic substance, a drug, a dye, a nutrient, an enzyme, a rheumatoid factor, a tumor marker, a microorganism, an opiate, a viral epitope, cholesterol, a heavy metal, a vitamin, an allergy, a neurodegenerative disorder, an electrolyte, a glycan, a polyamine, a fatty acid, a sugar, a chemical trace metals and mixtures thereof.

In another aspect the test sample is a target molecule selected from the group of emergent chemicals, organic contaminants, mold, fungus, asbestos, nitrogen compounds, pesticides, herbicides, sulfates, bacteria, methylcyclohexane methanol, polychlorinated biphenyls (PCBs) and mixtures thereof.

In one aspect the size of the at least one spot on the biochip can range from 1 μm to 4.0 mm in diameter.

The spots on the biochip are measured using a surface mass spectrometer or similar spectroscopic techniques. Examples of surface mass spectrometers or similar spectroscopic techniques include nano-Secondary Ion Mass Spectrometer (nano-SIMS), Time of Flight Secondary Ion Mass Spectrometer (TOF-SIMS), Time of Flight Matrix-Assisted Laser Desorption/Ionization (Maldi-Tof), electron spray ionization mass spectrometry (ESIMS), Ion Mobility Spectrometer (IMS), Secondary Ion Mass Spectrometer (SIMS), Matrix-Assisted laser Desorption/Ionization (MALDI), Surface Enhanced Laser Desorption/Ionization (SELDI), Surface-Assisted Laser Desorption/Ionization (SALDI), Auger Electron Spectroscopy (AES), Energy-Dispersive X-ray Spectroscopy (EDS or EDX), X-ray Photoelectron Spectroscopy (XPS) and Dynamic Secondary Ion Mass Spectrometer (D-SIMS).

In another aspect the present invention provides a kit for use in surface mass spectrometry or similar spectroscopic techniques for quantitating test samples comprising the biochip, as described herein, and a detection molecule, which is labeled with a detection signal source. The kit can also contain instructions for using the biochip and/or further assay reagents.

The detection molecule labeled with the detection signal source in the kit can be labeled with an element of I, Br, F, S, Au, Fe, Se, As, P, B, Cu, Ag, Zn, Ni or a lanthanide. Furthermore, the signal source is one of a group of atoms having a relative sensitivity factor of <1E23 under negative ion bombardment, or a relative sensitivity factor of <1E24 under positive ion bombardment.

In yet another aspect the biochip, as described herein, can be used for quantitating a target molecule in at least one assay spot.

A method of quantitating a target molecule in at least one assay spot is another embodiment of the present invention. This method comprises producing the biochip, as described herein, placing at least one target molecule and the detection molecule on designated places on said biochip allowing the at least one target molecule to interact with the capture molecule, detecting the presence of the at least one detection molecule on said biochip by counting detecting molecules using a surface mass spectrometer or similar spectroscopic techniques and calculating the quantity of said at least one target molecule on said biochip.

In this method a surface mass spectrometer or similar spectroscopic techniques, as described herein, can be used to detect the detection molecule.

The detection molecule, as described herein is labeled with an element of I, Br, F, S, Au, Fe, Se, As, P, B, Cu, Ag, Zn, Ni or a lanthanide having a relative sensitivity factor of <1E23 under a negative ion bombardment or a relative sensitivity factor of <1E24 under a positive ion bombardment.

The invention also relates to the following items (1 to 38), i.e.:

1. A biochip for use in surface mass spectrometry or similar spectroscopic techniques for quantitating a target molecule in a test sample comprising :

a substrate to which are attached at least one assay spot(s) containing capture molecules and comprising at least three control spots containing a signal control molecule each control spot having different concentrations of signal control molecules.

2. The biochip according to Item 1, wherein said capture and signal control molecules are attached to said substrate through a functionalized moiety.

3. The biochip according to Item 2, wherein said capture and signal control molecules are functionalized for attachment to said substrate.

4. The biochip according to any one of Items 1 to 3, wherein said capture and signal control molecules are attached to said substrate through non covalent bonds or covalent bonds.

5. The biochip according to Item 1, wherein the at least three control spots containing a signal control molecule further comprises a dilution molecule.

6. The biochip according to Item 5, wherein the amount of dilution molecule is present in the same amount as the capture molecule in the at least one assay spot.

7. The biochip according to any one of Items 1 to 5, wherein the signal control molecule is labeled with a first signal source.

8. The biochip according to any one of Items 5 to 7, wherein the dilution molecule is labeled with a second signal source.

9. The biochip according to Item 7, wherein the first signal source is iodine, bromine, fluorine, sulfur gold, iron, gold, iron, selenium, arsenic, lead, boron, silver, copper, zinc, nickel or a lanthanide.

10. The biochip according to Items 5 to 7, wherein the second signal source is different from the first signal source and is an element of iodine, bromine, fluorine, sulfur gold, iron, gold, iron, selenium, arsenic, lead, boron, silver, copper, zinc, nickel or a lanthanide.

11. The biochip according to any one of Items 1 to 10, wherein the capture molecules are attached onto said substrate and bind specifically with the target molecule.

12. The biochip according to any one of Items 1 to 11, wherein the signal control molecule does not bind to the target molecule and is attached onto the substrate.

13. The biochip according to any one of Items 1 to 11, wherein the capture molecules and signal control molecules are antibodies, nucleic acids, aptamers, affibodies, proteins, molecular imprinted polymers, enzymes, ligands, glycans, lectins, lipids, polyamines, phages, viruses, chemicals or combinations thereof

14. The biochip according to Item 13, wherein the capture molecules and signal control molecules are antibodies or nucleic acids.

15. The biochip according to any one of Items 1 to 14, wherein the substrate is fabricated from a material selected from the group of plastics, silicon, fused quartz, soda lime glass, ceramics, pyrex, metals, glassy carbon and polymeric materials.

16. The biochip according to Item 15, wherein said substrate is a silicon wafer.

17. The biochip according to any one of Item 15 or Item 16, wherein the solid substrate is in the shape of a rectangle, a square, a circle, a triangle, an oval, a pentagon, an octoagon or a hexagon.

18. The biochip according to any one of Items 1 to 17, wherein said spots on said biochip are microfabricated or nanofabricated.

19. The biochip according to any one of Items 1 to 18, wherein said at least one assay spot is a histological sample, a cytological sample, a biological sample or an environmental sample.

20. The biochip according to Item 19, wherein said histological sample is a tissue or a tumor or an organ or bone or skin.

21. The biochip according to Item 19, wherein said cytological sample is a cell, cell lysate or cell culture.

22. The biochip according to Item 19, wherein said biological sample is blood, serum, plasma, cerebrospinal fluid, seminal fluid, amniotic fluid, bile, gastric juices, saliva, tears, sweat, sputum, semen, peritoneal fluid, pericardial fluid, urine, feces, hair or nail clippings.

23. The biochip according to Item 19, wherein the environmental sample is air, water, waste water, food, soil, sand, ash, dust, mud, rocks, plants, trees, sludge, sediment, mining waste, pulp and paper waste or general manufacturing waste.

24. The biochip according to Item 1, wherein said at least one assay spot containing said capture molecules binds to a target molecule in a test sample.

25. The biochip according to Item 24, wherein said target molecule selected from the group of a protein, a peptide, a nucleic acid, a carbohydrates, a lipid, a polysaccharide, a glycoprotein, a hormone, an antigen, a pathogen, a toxic substance, a drug, a dye, a nutrient, an enzyme, a rheumatoid factor, a tumor marker, a microorganism, an opiate, a viral epitope, cholesterol, a heavy metal, a vitamin, an allergy, a neurodegenerative disorder, an electrolyte, a glycan, a polyamine, a fatty acid, a sugar, a chemical trace metals and mixtures thereof.

26. The biochip according to Item 25, wherein said test sample is a target molecule selected from the group of emergent chemicals, organic contaminants, mold, fungus, asbestos, nitrogen compounds, pesticides, herbicides, sulfates, bacteria, methylcyclohexane methanol, polychlorinated biphenyls (PCBs) and mixtures thereof.

27. The biochip according to any one of Items 1 to 26, wherein the size of the at least one spot range from 1 $\mu$m to 4.0 mm in diameter.

28. The biochip according to any one of Items 1 to 27, wherein the spots on said biochip are measured using a surface mass spectrometer or using similar spectroscopic techniques.

29. The biochip according to Item 28, wherein said surface mass spectrometer or similar spectroscopic techniques are selected from the group of nano-Secondary Ion Mass Spectrometer (nano-SIMS), Time of Flight Secondary Ion Mass Spectrometer (TOF-SIMS), Time of Flight Matrix-Assisted Laser Desorption/Ionization (Maldi-Tof), electron spray ionization mass spectrometry (ESIMS), Ion Mobility Spectrometer (IMS), Secondary Ion Mass Spectrometer (SIMS), Matrix-Assisted laser Desorption/Ionization (MALDI), Surface Enhanced Laser Desorption/Ionization (SELDI), Surface-Assisted Laser Desorption/Ionization (SALDI), Auger Electron Spectroscopy (AES), Energy-Dispersive X-ray Spectroscopy (EDS or EDX), X-ray Photoelectron Spectroscopy (XPS) and Dynamic Secondary Ion Mass Spectrometer (D-SIMS).

30. A kit for use in surface mass spectrometry for quantitating test samples comprising:

    (a) the biochip according to any one of Items 1 to 29; and
    (b) a detection molecule to which is attached a detection signal source.

31. A kit for use in surface mass spectrometry for quantitating test samples comprising:

    (a) the biochip according to any one of Items 1 to 29; and
    (b) reagents to graft detection a detection signal source to detection or target molecules.

32. A kit containing the biochip of any one of Items 1 to 29 and further comprising instructions for using the biochip.

33. The kit according to Item 30 or 31, further comprising assay reagents.

34. The kit according to any one of items 30 to 32, wherein said detection signal source is an element of I, Br, F, S, Au, Fe, Se, As, P, B, Cu, Ag, Zn, Ni or a lanthanide.

35. The kit according to any one of items 30 to 33, wherein the signal source is one of a group of atoms having a relative sensitivity factor of <1E23 under negative ion bombardment, or a relative sensitivity factor of <1E24 under positive ion bombardment.

36. A biochip according to any one of Items 1 to 29 for use in quantitating a target molecule in at least one assay spot.

37. A method of quantitating a target molecule in at least one assay sample comprising:

(a) producing the biochip according to any one of Items 1 to 27;
(b) placing at least one target molecule on designated places on said biochip allowing the at least one target molecule to interact with the capture molecule;
(c) placing at least one detection molecule on designated places on the biochip;
(d) detecting the presence of the at least one detection molecule on said biochip by counting detecting molecules using a surface mass spectrometer; and
(e) calculating the quantity of said at least one target molecule on said biochip.

38. The method according to Item 36, wherein said surface mass spectrometer or similar spectroscopic techniques are selected from the group of nano-Secondary Ion Mass Spectrometer (nano-SIMS), Time of Flight Secondary Ion Mass Spectrometer (TOF-SIMS), Time of Flight Matrix-Assisted Laser Desorption/Ionization (Maldi-Tof), electron spray ionization mass spectrometry (ESIMS), Ion Mobility Spectrometer (IMS), Secondary Ion Mass Spectrometer (SIMS), Matrix-Assisted laser Desorption/Ionization (MALDI), Surface Enhanced Laser Desorption/Ionization (SELDI), Surface-Assisted Laser Desorption/Ionization (SALDI), Auger Electron Spectroscopy (AES), Energy-Dispersive X-ray Spectroscopy (EDS or EDX), X-ray Photoelectron Spectroscopy (XPS) and Dynamic Secondary Ion Mass Spectrometer (D-SIMS).

## BRIEF DESCRIPTION OF THE DRAWINGS

Fig. 1 is a diagram of the disposition of different spots on a biochip.

Fig. 2 is a diagram of the different target proteins and their concentrations on the silicon wafer.

Fig. 3 is diagram of the results of a SIMS analysis to obtain the quantification of the entire spots on the biochip.

Fig. 4 is a diagram of the signal control curves that are correlated to normalize all the different wells on the biochip such that a direct comparison of the wells is possible.

Fig. 5 is a diagram of calibration curves of different target molecules in which the x-axis is the quantity of target molecules and the y-axis is the normalized SIMS signal.

Fig. 6 is a calibration curve showing the quantification of two target molecules based on the signal obtained and the calibration curves. The examples are TNF and II1.

Fig. 7 is a diagram showing the procedure of normalization after SIMS analysis to obtain the quantification of the entire spots on the biochip with the ratio method between iodine and bromine.

## DESCRIPTION OF THE PREFERRED EMBODIMENTS

As used herein "biochips" refers to a collection of microarrays arranged on a substrate that permits testing to be performed at the same time. The biochips can contain a defined set of capture molecules and signal control molecules that are immobilized at high density in a geometric pattern on the surface. The surface can be a solid planar surface or alternatively it can be a porous surface. It can also be in the form of a bead. The substrate can be fabricated from a material of plastic, silicon, fused quartz, soda lime glass, ceramics, pyrex, metals such as aluminum, titanium, stainless steel, glassy carbon and polymeric materials such as polystyrene and polycarbonate. Thus, the biochip can be in the form of glass slides, polyacrylamide gel pads, nitrocellulose membranes, beads, silicon slides or microtiter plates to which a target sample or detection molecule can be bound.
"Microfabricated" as used herein means that the substrate is fabricated with miniature structures on a micrometer scale or smaller using a microfabricating device such as pin arrayers, ink-jet printing or microfluidic devices. "Microfabricated" should be interpreted by the size of the spots comprised on the substrate.
"Nanofabricated" as used herein means that the substrate is fabricated with miniature structures on a nanometer scale or smaller using a nanofabricating device such as pin arrayers, ink-jet printing or microfluidic devices. "Nanofabricated" should be interpreted by the size of the spots comprised on the substrate.
As used herein the term "spot" means any mark on the biochip on which the capture molecules and signal control molecules or detection molecules are placed. Each "spot" occupies an individual and unique place on the biochip and in most cases there are no overlapping spots. The "spot" can be in any geometric shape such as rectangular, triangular, oval, hexagonal, pentagonal, square or in the shape of a circle. The size of the "spot" may vary depending upon the application.

The term "test sample," as described herein, means any sample that is fixed as a point of examination. The test sample contains the target molecules that are to be quantified. In this regard the test sample can be derived from a histological sample, a cytological sample, a biological sample or an environmental sample. Examples of histological samples include, but are not limited to, tissues, tumors, organs, bone or skin. The test sample can be a lysate of a tissue, a lysate of a tumor, a lysate of an organ or a lysate of skin. Examples of cytological samples include, but are not limited to, cells, cell lysates or cell culture. Examples of biological samples include, but are not limited to, blood, serum, plasma, cerebrospinal fluid, seminal fluid, amniotic fluid, bile, gastric juices, saliva, tears, sweat, sputum, semen, peritoneal fluid, pericardial fluid, urine, feces, hair, nail clippings and the like. Air, water, food, mining waste, pulp and paper waste or general manufacturing waste and the like are examples of environmental samples. The samples can be obtained from any animal and especially mammals or any environmental sample.

Examples of target molecules that can be quantified include proteins, peptides, nucleic acids, carbohydrates, lipids, polysaccharides, glycoproteins, hormones, antigens, antibodies, pathogens such as viruses, bacterium, or fungus, toxic substances, drugs, dyes, nutrients, enzymes, rheumatoid factor, tumor markers, microorganisms, drugs, opiates, viral epitopes, cholesterol, heavy metals, vitamins, allergies, neurodegenerative disorders, electrolytes, glycans, polyamines, fatty acids, sugars, allergens, chemicals trace metals, organic contaminants, mercury, dioxin, emergent chemicals, polychlorinates biphenyls and the like.

"Detecting" means quantitating the amount of target molecule present in the sample. The detection can be performed using a surface mass spectrometer or similar spectroscopic techniques. Examples of surface mass spectrometers or similar spectroscopic techniques include nano-Secondary Ion Mass Spectrometer (nano-SIMS), Time of Flight Secondary Ion Mass Spectrometer (TOF-SIMS), Time of Flight Matrix-Assisted Laser Desorption/Ionization (Maldi-Tof), electron spray ionization mass spectrometry (ESIMS), Ion Mobility Spectrometer (IMS), Secondary Ion Mass Spectrometer (SIMS), Matrix-Assisted laser Desorption/Ionization (MALDI), Surface Enhanced Laser Desorption/Ionization (SELDI), Surface-Assisted Laser Desorption/Ionization (SALDI), Auger Electron Spectroscopy (AES), Energy-Dispersive X-ray Spectroscopy (EDS or EDX), X-ray Photoelectron Spectroscopy (XPS) and Dynamic Secondary Ion Mass Spectrometer (D-SIMS). In one aspect Dynamic Secondary Ion Mass Spectrometer (D-SIMS) is used.

"Similar spectroscopic techniques" means techniques that are used to analyze the compositions of surfaces or ion mobility. Examples include electron spray ionization mass spectrometry (ESIMS), Ion Mobility Spectrometer (IMS), Surface Enhanced Laser Desorption/Ionization (SELDI), Surface-Assisted Laser Desorption/Ionization (SALDI), Auger Electron Spectroscopy (AES), Energy-Dispersive X-ray Spectroscopy (EDS or EDX) and X-ray Photoelectron Spectroscopy (XPS).

As used herein "detection molecules" are molecules used for the detection of the target molecule. Detecting molecules are molecules that specifically bind with the target molecules or are grafted with the sample before putting the sample on the biochip. The detection molecule is labeled with a detection signal source by methods known in the art. Examples of signal sources that can be used to label the detection molecule is an element that includes iodine, bromine, fluorine, sulfur, gold, iron, gold, iron, selenium, arsenic, lead, boron, silver, copper, zinc, nickel or a lanthanide. If more than one detecting molecule is used to target more than one target molecule on the biochip, the signal sources may vary. For instance, to target molecule X, iodine can be used as a signal source and to target molecule Y gold can be used as a signal source.

"Capture molecules," as used herein, refer to molecules such as ligands, receptors, aptamers, DNA segments, enzymes, antigens, antibodies and the like which are used to specifically bind the target molecules in the test sample.

"Signal control molecules," as used herein, are molecules that do not bind with the target molecule and are labeled with a signal source. The "signal control molecules" are attached to the substrate through a functionalized moiety. This functionalized moiety may be a functional groups or linkers, as described herein, and the attachment can be through nonspecific and noncovalent immobilization, nonspecific and covalent immobolization, specific and noncovalent immobilization or specific and covalent immobilization.

The "signal control molecules" are attached to the surface of the microarray such that they remain on the microarray even after washing. The "signal control molecules" comprise at least three spots on the biochip and each spot has a different concentration of molecules, which are used to create a curve corresponding to the signal response in function of the amount of detection molecules that is sought in the assay spots after the biochip is analyzed. All of the signal responses of each of the target molecules for one microarray are compared to the specific curve generated by the "signal control molecules" to measure the relative quantity in each sample. All of the curves given by the "signal control molecules" are compared between arrays and normalized to compare one sample with the others.

"Signal source" as used herein means any element or a specific group of elements that can be used to detect a signal control molecule or a detection molecule or a capture molecule or dilution molecule to which the signal is attached using a surface mass spectrometer or similar spectroscopic techniques.

As used herein, "dilution molecule" means any molecule that reduces the concentration of the signal control molecules. The "dilution molecule" utilized depends on the capture molecules utilized and varies according to the "family" of the capture molecules that are used on the biochip. For instance, if an antibody is used as the capture molecules, the dilution molecule is preferably an antibody. If an aptamer is used as a capture molecule, an aptamer is preferably used as a "dilution molecule." However, different molecules are used in the "dilution molecule" from those of the capture molecule. In one aspect the amount of "dilution molecule" in the control spots is the same as the amount of capture molecule in the assay spots.

As used herein, the term "kit" refers to any delivery system for delivering materials. In the context of reaction assays, such delivery systems include the biochip and/or supporting materials (e.g., buffers, written instructions for performing the assay) from one location to another. For example, kits include one or more enclosures (e.g., boxes) containing the biochip and/or relevant reaction reagents. As used herein, the term "fragmented kit" refers to delivery systems comprising two or more separate containers that each contain a subportion of the total kit components. The containers may be delivered to the intended recipient together or separately. For example, a first container may contain the biochip for use in an assay, while a second container contains relevant reaction agents.

By "consisting essentially of" means that the major elements are present in the biochip, but also minor ingredients that do not materially affect the analysis of the biochip can also be present.

The present invention thus relates to a biochip for use in surface mass spectrometry or similar spectroscopic techniques for quantitating a target molecule in a test sample comprising a substrate to which are attached at least one assay spot(s) containing capture molecules and comprising at least three control spots containing a signal control molecules each control spot having different concentrations of signal control molecules.

The surface of the biochip, as described herein, is a substrate to which the at least one spot(s) containing the capture molecules and the at least three spots containing the signal control molecules can be attached. The attachment of the at least one spot(s) containing the capture molecules and the at least three spots containing the signal control molecules to the substrate can be by any means known in the art such as by nonspecific and noncovalent immobilization, nonspecific and covalent immobilization, specific and noncovalent immobilization or specific and covalent immobilization.

Nonspecific and noncovalent immobilization relies on physical adsorption of proteins or molecules to substrates. As a general rule, essentially all proteins will adsorb to essentially all surfaces. Physical adsorption can be to substrates such as nitrocellulose, polystyrenes or silanized glass. The at least one assay spot containing capture molecules and the at least three control spots can be adsorbed to the surface of the substrate by applying a drop of buffer containing these molecules to the surface of the substrate. In this embodiment the capture molecules and signal control molecules are attached to the substrate through non covalent bonds.

Nonspecific and covalent immobilization requires substrates that have functional groups that can be used to covalently link the molecule to the chip to avoid the exchange of immobilized proteins, and can be applied to low-molecular-weight molecules including peptides. Thus the functionalized moiety to which the capture molecules and signal control molecules are attached to the substrate include functional groups that can be used to immobilize the target sample or detection molecule to the substrate include, but are not limited to, amino groups, aldehyde groups, carboxyl groups, sulfhydryl groups, phenyl groups, benzyl groups, hydroxyl groups, carbonyl groups, imide groups, thiol groups, phosphate groups, epoxy silane groups and the like. In one aspect expoxy silane groups are used to functionalize the substrate.

Examples of functionalized moieties containing functional groups that can be used to immobilize the capture molecules and signal control molecules include glass slides modified with N-hydroxysuccinimide (NHS) esters or with aldehydes, cyano, amino/bifunctional N-hydroxysuccinmde, mercapto or expoxy functional groups that are covalently bonded to amino groups, hydroxyl groups or thiol groups. In one embodiment the substrate is functionalized with epoxysilane.

Specific and noncovalent immobilization includes the use of substrates that present molecular groups that can selectively interact with a tag on the protein. Attachment of biotin-tagged ligands to substrates that are modified with a layer of streptavidin is the most common example of a specific but noncovalent strategy for immobilizing molecules as described by Yam et al J. Colloid. Interf Sci 296:118-130(2006).

An example of specific and covalent immobilization is the immobilization domain of a fusion protein was made to interact with an irreversible inhibitor, leading to a selective and covalent attachment of the protein to the substrate such as the binding of a serine esterase cutinase to a self-assembled monolayer presenting a phophonate ligand as described by Hodneland et al, PNAS vol. 99 No. 8 5048-5052 (2002).

Processes for attachment of the capture molecules or signal control molecules to the substrate that can be used include expressed protein ligation as described in Camarero et al, J. Am Chem Soc 2004:126(45)14730-1, click chemistry as described by Sun et al Bioconjugate Chem (2006) 17 52-57 or trans-splicing as described by Kwon et al Angew Chem Int Ed 2006:45(11)1726-9.

The substrate can also be layered with a polymer or different polymers such as polyallylamine, polyethylene terephthalate, polyethylene glycol, polyacrylamide or agarose. If the substrate is a polymer or layered with a polymer it can also be coated with a layer of carbon or diamond-like carbon by spattering, chemical vapor deposition (CVD) or physical vapor deposition (PVD) to ensure covalent bonds with the polymer.

Besides functionalizing the substrate the capture molecules and signal control molecules can be functionalized with the functional groups, as described herein, for their attachment to the substrate. Linkers can also be used to attach the functionalizing group on the substrate or capture molecules or signal control molecules. The linkers can be short peptide sequences or bifunctional chemical linkers.

Peptide linkers are often composed of flexible residues like glycine and serine so that the adjacent protein domains are free to move relative to one another. Longer linkers are used when it is necessary to ensure that two adjacent domains do not sterically interfere with one another.

Bifunctional chemical linkers, include, for example, 3-maleimidobenzoic acid N-hydroxyysuccinimide ester (MBS), N-succinimidyl 3-[2-pyridyldithio]-propionate (SPDP), [N-Succinimidyl)-S-acetylthioacetate] (SATA), [(N-succinimidiyloxy carbonyl)1-methyl-1-(2-pyridyldithio) toluene] (SMPT), 2-iminothiolane (2-IT), (Sulfosuccinimidyl N-[3-(Acetylthio)-3-methylbutyryl)-beta-alanine]) (sulfoNHS-ATMBA), thioimidates such as AMPT and M-CDPT and 2-[N-chlorocarbonyl)-N-methylamino]1-ethyl 2-pyridyldisulfide can be used.

In one aspect the signal control molecules having at least three control spots each spot having a different concentration of the signal control molecules can be diluted with a dilution molecule. The dilution molecule is of the same "family" as the capture molecules; i.e., if the capture molecule is an antibody, the dilution molecule is also an antibody. However, the dilution molecule is different from the capture molecule and does not bind the target molecule. The dilution molecule can be present in any amounts depending on the type of signal control molecules that are used on the biochip. In one aspect the dilution molecule is present in the same amount as the capture molecule. In another aspect the dilution molecule is present in an amount that is ten times the amount of the capture molecule. In yet another aspect, the dilution molecule is present in amount of 0.1 % of the amount of the capture molecule.

The signal control molecules are labeled with a first signal source, do not bind to the target molecule and are attached to the substrate, as described herein. This first signal source is an element of iodine, bromine, fluorine, sulfur gold, iron, gold, iron, selenium, arsenic, lead, boron, silver, copper, zinc, nickel or a lanthanide.

The signal control molecule, detection molecule, capture molecules and dilution molecules can be labeled with the elements, described herein, using methods known in the art. These methods include chemical methods such as chloramine T or derivatives thereof, click chemistry (Kolodych et al., Angewandte Chemie International Edition Volume 52, Issue 46 Pgs. 12056-12060, (November 2013)), PEGylation by linking to a protein or peptide through reactive molecular groups on amino acid side chains; i.e., most often lysine, chemical synthesis and the like. In another aspect of the invention includes the use of binding moieties such as biotin and streptavidin.

The range of concentration of the signal control molecules may vary with the type of molecules that are used on the biochip, as well as the type of assay. In one aspect the signal control molecules have a range of concentration that mimics the amount of detection molecules that is sought in the assay spots after the biochip is assayed with standard samples and detection molecules. In one aspect, the signal control molecule is present in the same amount as the capture molecule in a first spot; at 50% of the amount of the capture molecule in a second spot; and at 25% of the amount of the capture molecule in a third spot. In another aspect, the signal control molecule is present in the same amount as the capture molecule in a first spot; at 10% of the amount of the capture molecule in a second spot; and at 0.1 % of the amount of the capture molecule in a third spot.

In another aspect the dilution molecule can also be labeled with a signal source. This signal source is a different signal source than that of the signal control molecules and is an element of iodine, bromine, fluorine, sulfur gold, iron, gold, iron, selenium, arsenic, lead, boron, silver, copper, zinc, nickel or a lanthanide.

The capture molecules and signal control molecules can be antibodies, nucleic acids, aptamers, affibodies, proteins, molecular imprinted polymers, enzymes, ligands, glycans, lectins, lipids, polyamines, phages, viruses, chemicals or combinations thereof. In one aspect the capture molecules and signal control molecules are antibodies. In another aspect, the capture molecules and signal control molecules are nucleic acids.

The substrate can have a rigid or semi-rigid surface. At least one surface of the substrate can be essentially flat in one embodiment. The substrate or solid substrate can in the shape of a rectangle, a square, a circle, a triangle, an oval, a pentagon, an octoagon or a hexagon. The size and shape of the substrate may vary depending on the size of the sample chamber in the surface mass spectrometer or similar spectroscopic techniques used and its application. For instance, they can range from 1 cm x 2 cm, 2.5 cm x 2.5 cm, 2 cm x 3 cm, 6 cm x 12 cm or 6 mm x 6 mm for slides.

In another embodiment to physically separate the capture molecules and the signal control molecules the substrate can have wells, raised regions, etched trenches and the like. The substrate can be made of any material such as plastic, silicon, fused quartz, glass, soda lime glass, ceramics, pyrex, metals such as aluminum, titanium, stainless steel, glassy carbon and polymeric materials such as polystyrene, polycarbonate. In one embodiment the substrate is made of a silicon wafer.

Prior to use the substrate can be washed using, for example, organic solvents, methylene chloride, dimethylformamide (DMF), ethyl alcohol or the like. The solvents will depend on the material of the substrate chosen and are well known in the art.

The spots can be any geometric shape such as square, rectangular, triangular hexagonal, oval, octagonal or hexagonal. The size of the spot may vary depending on the assay being performed and the substrate or solid substrate used. The size of the spot can range from 1 $\mu$m to 4.0 mm. In another embodiment the spots can range from 5.0 $\mu$m to 500 $\mu$m or 10 $\mu$m to 200 $\mu$m or 50 $\mu$m to 100 $\mu$m.

A typical biochip, as described herein, can have between 4 to 200,000 spots. In another embodiment the number of spots can range from 4 to 5,000 spots. In yet another embodiment the number of spots can range from 4 to 500. Thus the term "at least one" encompasses one spot, but can also encompass 400 spots or 1,000 spots or 8,000 spots. The term at least three spots can encompass three spots or 450 spots or 20,000 spots.

The capture molecules, signal control molecules and dilution molecules are each present in the spot in a range from 1 molecule per spot to $10^{10}$ molecules per spot.

The biochip, as described herein, contains at least one assay spot containing capture molecules and at least three control spots containing signal control molecules.

The spots can be arranged in any manner on the biochip. The spots are generally arranged in rows for clearer scanning.

The spots can be generated at high density using spot arrayers such as those described in U.S. Patent 6,040,193 or U.S. Patent 5,885,837, incorporated herein by reference. Thus, the spots contain the reactive regions while in between the spots are inert regions. The spots can be generated, for example, with pin arrayers, nanoarrayers, microarrayers, ink-jet printing devices, microfluid devices, piezo electric pumps, SpotBot3® microarrayer, a SciFlex arrayer and the like. Split pins or solid pins can be used as the pin head in these arrayers. These devices are well known in the art.

The biochip, as described herein can be microfabricated or nanofabricated using specific types of printers.

The at least one assay spot can be a histological sample, a cytological sample, a biological sample or an environmental sample.

The histological sample can be a tissue or a tumor or an organ or skin. The histological sample can be a lysate of a tissue or a lysate of tumor or a lysate of an organ or a lysate of skin. In immunohistochemistry antigens in cells of tissue are detected by using antibodies that specifically bind to the antigen. The tissue, tumor, organ or skin is collected, fixed and sectioned. Fixation of tissues can be accomplished by chemical or physical means. Physical means can include micro-waving, heating or cryo-preservation. Chemical fixation is accomplished by immersing the specimen in a fixative. Fixative solutions for fixing the tissue are well known in the art and vary from the type of tissue being tested. Therefore, examples of fixative include 10% or 20% buffered formalin, Boulin solution (picric acid, acetic acid, formalin), Carnoy mixture (absolute ethanol, chloroform, acetic acid, formalin), Davidson fixing agent (paraformaldehyde in phosphate buffer), Helly mixture (corrosive sublimate, potassium dichromate), Susa solution (corrosive sublimate, trichloroacetic acid, sodium chloride acetic acid, formalin) or Zenker solution (corrosive sublimate potassium dichromate, acetic acid).

Before sectioning the tissue sample may be embedded in a medium like paraffin wax or cryomedia. Sections can be sliced on a variety of instruments such as microtomes or cryostats and are generally sliced to a size of between 4 to 40 $\mu$m. The slices can be dehydrated using alcohol after they are mounted on slides and cleared using, for example xylene.

Depending on the method of fixation and tissue preservation of the sample additional steps such as deparaffinization and antigen retrieval are undertaken. The antigen retrieval method may vary depending on whether chemical or physical fixation was undertaken. These methods are well known in the art.

The target molecule can be an antigen, which can be detected by direct methods which involves a sole antibody that is labeled that reacts directly with the antigen in tissues or a an indirect method that uses an unlabeled primary antibody that binds to the test sample antigen in the tissue and a labeled secondary antibody that reacts with the primary antibody.

Alternatively, the target molecule can be directly labelled with the signal source, using methods known in the art. These methods include chemical methods such as chloramine T or derivatives thereof, click chemistry (Kolodych et al., Angewandte Chemie International Edition Volume 52, Issue 46 Pgs. 12056-12060, (November 2013)), PEGylation by linking to a protein or peptide through reactive molecular groups on amino acid side chains; i.e., most often lysine, chemical synthesis and the like. In another aspect the invention includes the use of binding moieties such as biotin and streptavidin.

In one aspect a solid tumor can be biopsied from the patient, subjected to immunohistochemical preparation, as described herein, such as fixing in formalin and embedding in paraffin the tumor sections. The tumor specimens are then immobilized on a substrate such as a silicon wafer. Primary antibodies against tumor antibodies are labeled with highly pure isotopic metals such as stable lanthanides (atomic numbers 58 to 71). The primary antibodies are then combined in solution and incubated with the specimen and mounted in a sample holder in a multiplexed ion beam imagining scanner. The specimen is then subjected to a rasterized oxygen duoplasmatorn primary ion beam. This ion beam liberates secondary ions of the bound antibodies when the lanthanide is struck. The secondary ions are then measures in a magnetic mass spectrometer.

In the above tumor embodiments, breast tumors can be measured using breast tumor antibodies directed against ER alpha, PR, Ki67, Vimentin. E-cadherin, Pan-keratin Her2 Pan-actin and the like which are labeled with elemental isotopes such as $^{141}Pr$, $^{142}Nd$, $^{143}Nd$, $^{144}Nd$, $^{145}Nd$, $^{146}Nd$, $^{147}Sm$, $^{148}Nd$, $^{149}Sm$, $^{150}Nd$, $^{151}Eu$, $^{152}Sm$, $^{153}Eu$, $^{154}Sm$, $^{156}Gd$, $^{158}Gd$, $^{159}Tb$, $^{160}Gd$, $^{161}Dy$, $^{162}Dy$, $^{163}Dy$, $^{164}Dy$, $^{165}Ho$, $^{166}Er$, $^{167}Er$, $^{168}Er$, $^{169}Tm$, $^{170}Er$, $^{171}Yb$, $^{172}Yb$, $^{173}Yb$, $^{174}Yb$, $^{175}Lu$, $^{176}Yb$.

In another aspect colorectal tumors can be subjected to immunohistochemistry in a similar manner as breast tumors. Tumor antibodies directed against CA19-9, CA125, CEA, MSI and the Kras mutation can be used in this analysis, as well as the elemental isotopes set forth above ; i.e., $^{141}PR$, $^{142}Nd$, $^{143}Nd$, $^{144}Nd$, $^{145}Nd$, $^{146}Nd$, $^{147}Sm$, $^{148}Nd$, $^{149}Sm$, $^{150}Nd$, $^{151}Eu$, $^{152}Sm$, $^{153}Eu$, $^{154}Sm$, $^{156}Gd$, $^{158}Gd$, $^{159}Tb$, $^{160}Gd$, $^{161}Dy$, $^{162}Dy$, $^{163}Dy$, $^{164}Dy$, $^{165}Ho$, $^{166}Er$, $^{167}Er$, $^{168}Er$, $^{169}Tm$, $^{170}Er$, $^{171}Yb$, $^{172}Yb$, $^{173}Yb$, $^{174}Yb$, $^{175}Lu$, $^{176}Yb$.

If the test sample is being cytologically examined the cytological sample can be a cell, cell lysate or cell culture. Cells are thus collected from the patient and analyzed or can be subjected to cell culture. The cell culture or collected cells can be further lysed if necessary.

Depending on the type of analysis, the cells can be derived from various parts of the patient. Indeed, the cells can be collected from the female reproductive tract, ureters, urinary bladder and urethra, peritoneum, pleura, pericardium, breast, thyroid gland, lymph nodes, lung and airways, alimentary tract, soft tissue, bone skin, kidney, adrenal gland, liver pancreas, central nervous system, eye, saliva, sputum, mucous, bone marrow, mononuclear cells and epithelia cells.

The cells can be collected by swabbing the desired area, scraping the desired area or by needle aspiration biopsy. The sample can be subjected to sediment cytology.

In one aspect anti- epidermal growth factor (EGFR) antibodies are spotted on the biochip as the capture molecule, as described herein, and circulating tumor cells are used as the test assay to detect cancer by the quantification of EGFR (target molecule) with single ion mass spectrometry (SIMS).

The biological sample can also be blood, serum, plasma, cerebrospinal fluid, seminal fluid, amniotic fluid, bile, gastric juices, saliva, tears, sweat, sputum, semen, peritoneal fluid, pericardial fluid, urine, feces or nail clippings. These test samples can be collected by methods known in the art and can be used for quantifying target molecules present in the test samples such as proteins, peptides, nucleic acids, carbohydrates, lipids, polysaccharides, glycoproteins, hormones, antigens, pathogens such as viruses, bacterium, or fungus, toxic substances, drugs, dyes, nutrients, enzymes, rheumatoid factor, tumor markers, microorganisms, drugs, opiates, viral epitopes, cholesterol, heavy metals, vitamins, allergies, neurodegenerative disorders, electrolytes, glycans, polyamines, fatty acids, sugars, chemical trace metals, organic contaminants, mercury, dioxin, emergent chemicals and/or polychlorinates biphenyls and the like. Scanners, as described herein, are used in this quantification.

In another aspect the test sample is an environmental sample. This environmental sample can be air, water, waste water, soil, sand, ash, dust, mud, rocks, plants, trees, sediments, sludge, organic contaminants, food, mining waste, pulp and paper waste or general manufacturing waste. The target molecule in the environmental test sample can be mercury, dioxin, emergent chemicals, mercury, trace metals, organic contaminants, mold, fungus, radon, lead, iron, copper, asbestos, nitrogen compounds, pesticides, herbicides, sulfates, bacteria, methylcyclohexane methanol and/or polychlorinated biphenyls (PCBs).

The target molecule can be selected from the group of a protein, a peptide, a nucleic acid, a carbohydrates, a lipid, a polysaccharide, a glycoprotein, a hormone, an antigen, a pathogen, a toxic substance, a drug, a dye, a nutrient, an enzyme, a rheumatoid factor, a tumor marker, a microorganism, an opiate, a viral epitope, cholesterol, a heavy metal, a vitamin, an allergy, a neurodegenerative disorder, an electrolyte, a glycan, a polyamine, a fatty acid, a sugar, a chemical trace metals and mixtures thereof.

In another aspect the target molecule can be selected from the group of mercury, dioxin, emergent chemicals, mercury, trace metals, organic contaminants, mold, fungus, radon, lead, iron, copper, asbestos, nitrogen compounds, pesticides, herbicides, sulfates, bacteria, methylcyclohexane methanol, polychlorinated biphenyls (PCBs) and mixtures thereof.

The amount of target molecule used in the assay will vary with the type of target molecule that is to be quantified. Thus, for example cytokines would have amounts ranging from 1 fg/ml to 1 ng/ml, while albumin would range from 1,100 μg/ml to 10 mg/ml.

The biochip, as described herein, can be measured using a surface mass spectrometer or similar spectroscopic techniques. Examples of surface mass spectrometer or similar spectroscopic techniques include nano-Secondary Ion Mass Spectrometer (nano-SIMS), Time of Flight Secondary Ion Mass Spectrometer (TOF-SIMS), Time of Flight Matrix-Assisted Laser Desorption/Ionization (Maldi-Tof), electron spray ionization mass spectrometry (ESIMS), Ion Mobility Spectrometer (IMS), Secondary Ion Mass Spectrometer (SIMS), Matrix-Assisted laser Desorption/Ionization (MALDI), Surface Enhanced Laser Desorption/Ionization (SELDI), Surface-Assisted Laser Desorption/Ionization (SALDI), Auger Electron Spectroscopy (AES), Energy-Dispersive X-ray Spectroscopy (EDS or EDX), X-ray Photoelectron Spectroscopy (XPS) and Dynamic Secondary Ion Mass Spectrometer (D-SIMS).

In one aspect Dynamic Secondary Ion Mass Spectrometer (D-SIMS) is used to quantify target molecules.

In another aspect the present invention provides a kit for use in surface mass spectrometry or similar spectroscopic techniques for quantitating test samples comprising the biochip, as described herein and a labeled detection molecule to which is attached a signal source. This kit may further comprise instructions for using the biochip and/or assay reagents.

The detection molecule in the kit is labeled with a signal source. This signal source can be I, Br, F, S. Au, Fe, Se, As, P, B, Cu, Ag, Zn or Ni or a lanthanide. The signal source can be attached to the detection molecule via methods known in the art, as described herein.

In one aspect the signal source is one of a group of atoms having a relative sensitivity factor of <1E23 under negative ion bombardment, or a relative sensitivity factor of <1E24 under positive ion bombardment.

Relative sensitivity factors can be defined by the equation:

$$\frac{I_R}{C_R} = RSF_E = \frac{I_E}{C_E}$$

where $RSF_E$ is the Relative Sensitivity Factor for Element E, $I_E$ is the secondary ion intensity for Element E, $I_R$ is the secondary ion intensity for reference element R, $C_E$ is the concentration of E and $C_R$ is the concentration of R.

The major (or matrix) element is chosen as the reference and M is substituted for R in the equation which is rearranged as follows:

$$C_E = RSF_E = \frac{I_E C_M}{I_M}$$

RSF and CE have the same concentration units.

In yet another aspect a biochip, as described herein, is provided for use in quantitating a target molecule in at least one assay spot.

In yet another aspect the present invention provides a method of quantitating a target molecule in at least one assay sample comprising producing the biochip, as described herein, placing at least one target molecule on designated places on said biochip allowing the at least one target molecule to interact with the capture molecule; placing at least one detection molecule on designated places on the biochip; detecting the presence of the at least one detection molecule on said biochip by counting detecting molecules using a surface mass spectrometer; and calculating the quantity of said at least one target molecule on said biochip.

In this method the surface mass spectrometer or similar spectroscopic techniques are used such as nano-Secondary Ion Mass Spectrometer (nano-SIMS), Time of Flight Secondary Ion Mass Spectrometer (TOF-SIMS), Time of Flight Matrix-Assisted Laser Desorption/Ionization (Maldi-Tof), electron spray ionization mass spectrometry (ESIMS), Ion Mobility Spectrometer (IMS), Secondary Ion Mass Spectrometer (SIMS), Matrix-Assisted laser Desorption/Ionization (MALDI), Surface Enhanced Laser Desorption/Ionization (SELDI), Surface-Assisted Laser Desorption/Ionization (SALDI), Auger Electron Spectroscopy (AES), Energy-Dispersive X-ray Spectroscopy (EDS or EDX), X-ray Photoelectron Spectroscopy (XPS) and Dynamic Secondary Ion Mass Spectrometer (D-SIMS).

In one aspect Dynamic Secondary Ion Mass Spectrometer (D-SIMS) is used.

The detection molecule, as described herein, is labeled with an element of I, Br, F, S, Au, Fe, Se, As, P, B, Cu, Ag, Zn, Ni or a lanthanide having a relative sensitivity factor of <1E23 under a negative ion bombardment or a relative sensitivity factor of <1E24 under a positive ion bombardment.

A number of embodiments and/or aspects of the invention have been described.

Nevertheless it will be understood that various modifications may be made without departing from the spirit and scope of the invention.

**EXAMPLES**

**Example 1: Realization of a Biochip Containing Signal Control Molecules and Capture Molecules**

A. Materials: The materials that were used to fabricate the biochip were the following:

(1) Silicon wafers (18 mm x 18 mm) (Siltronix) were functionalized with an epoxysilane coating (Sigma). There are 16 different zones (4x4) on the wafer with a size of 4 mm x 4 mm. These zones are called wells.

(2) Capture Molecules: antibodies for the following target molecules were used:

    o Anti IL1 alpha (R&D Systems MAB200)
    o Anti-IL6 (R&D Systems MAB206)
    o Anti-IL-10 (R&D Systems MAB2172)
    o Anti-IL 17 (R&D Systems MAB1248)
    o Anti-TNF (R&D Systems MAB610)

(3) Signal Control Molecule : An antibody labelled with a click chemistry coupled NHS-PEG-Azide/Alkyne, 2-iodo-benzene

    a. Non-specific control (AbD Serotec HCA096): non relevant antibody

(4) Dilution molecule: antibody unlabelled

    a. Non-specific control (AbD Serotec HCA096): non relevant antibody

(5) Protein Printing Buffer 2X, Nexterion®
(6) BSA fraction V (Sigma Aldrich)
(7) PBS buffer

B. The biochips were formulated in the following manner:
In a 384 well plate, the different antibodies were diluted in protein printing buffer 2X as follows:

    o Well A1 : 10µL of antibody anti IL1 at 1 mg/mL + 10µL of protein printing buffer 2X
    o Well A2 : 10µL of antibody anti IL6 at 1 mg/mL + 10µL of protein printing buffer 2X
    o Well A3 : 10µL of antibody anti IL10 at 1 mg/mL + 10µL of protein printing buffer 2X
    o Well A4 : 10µL of antibody anti IL17 at 1 mg/mL + 10µL of protein printing buffer 2X
    o Well A5 : 10µL of antibody anti TNF at 1mg/mL + 10µL of protein printing buffer 2X
    o Well A6 : 10µL of labelled non-specific control at 1 mg/mL + 10µL of protein printing buffer 2X
    o Well A7 : 5µL of non-specific control at 1 mg/mL + 5µL of labelled non-specific control at 1 mg/mL + 10µL of protein printing buffer 2X
    o Well A8 : 7.5µL of non-specific control at 1 mg/mL + 2.5µL of labelled non-specific control at 1 mg/mL + 10µL of protein printing buffer 2X
    o Well A9 : 10µL of antibody non-specific control at 1 mg/mL + 10µL of protein printing buffer 2X

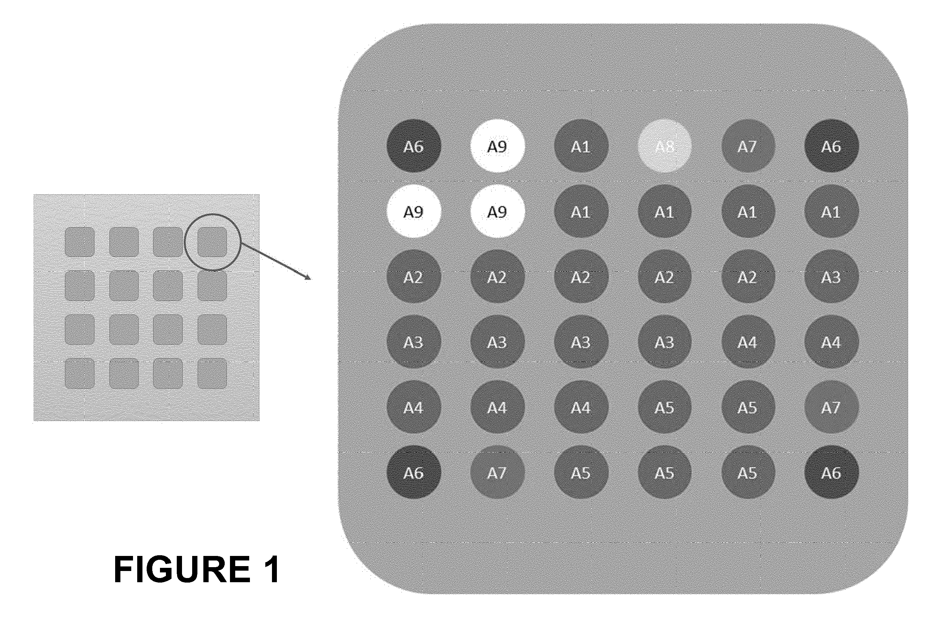

The well plate and the silicon wafers were placed in a SciFLEXARRAYER (Scienion). The arrayer, after being programmed appropriately, fabricated the arrays following Figure 1. The printed arrays were stored at 4°C overnight and then stored under vacuum at room temperature.

**Example 2: Test of Quantification of the Target Molecules in a Biological Sample**

A. Materials: The materials that were used in this example were the following:

(1) One Array, as described in Example 1.
(2) Recombinant Molecules: The target molecules used for calibration curve were the following:

    a. IL1 alpha (R&D Systems 200-LA-002)
    b. IL6 (R&D Systems 206-IL-010)
    c. IL-10 (R&D Systems 217-IL-005)
    d. IL 17 (R&D Systems 317-ILB-005)
    e. TNF alpha (R&D Systems 210-TA-005)

(3) Detection molecules: The detection molecules used were antibodies for the same target molecules labelled with a click chemistry coupled NHS-PEG-Azide/Alkyne, 2-iodo-benzene at 1µg/ml and were the following:

a. Anti IL1 alpha (R&D Systems AF-200-NA)
b. Anti-IL6 (R&D Systems AF-206-NA)
c. Anti-IL-10 (R&D Systems AF-217-NA)
d. Anti-IL 17 (R&D Systems AF-317-NA)
e. Anti-TNF alpha (R&D Systems AF-210-NA)

(4) BSA fraction V (Sigma Aldrich)
(5) PBS buffer
(6) Biological sample : plasma of a rheumatoid arthritis patient

The hybridization was carried out as follows. The wells were first blocked with a solution containing 1% BSA in a 1X PBS buffer. After blocking the different wells of the wafer were incubated 2H with 10μL of different test samples containing various quantities of the following target molecules:

a. Recombinant Target molecules (IL1, IL6, IL10 IL17 & TNF) at 0 pg/mL
b. Recombinant Target molecules (IL1, IL6, IL10 IL17 & TNF) at 0.1 pg/mL
c. Recombinant Target molecules (IL1, IL6, IL10 IL17 & TNF) at 1 pg/mL
d. Recombinant Target molecules (IL1, IL6, IL10 IL17 & TNF) at 5 pg/mL
e. Recombinant Target molecules (IL1, IL6, IL10 IL17 & TNF) at 25 pg/mL
f. Recombinant Target molecules (IL1, IL6, IL10 IL17 & TNF) at 50 pg/mL
g. Recombinant Target molecules (IL1, IL6, IL10 IL17 & TNF) at 100 pg/mL
h. Biological Sample

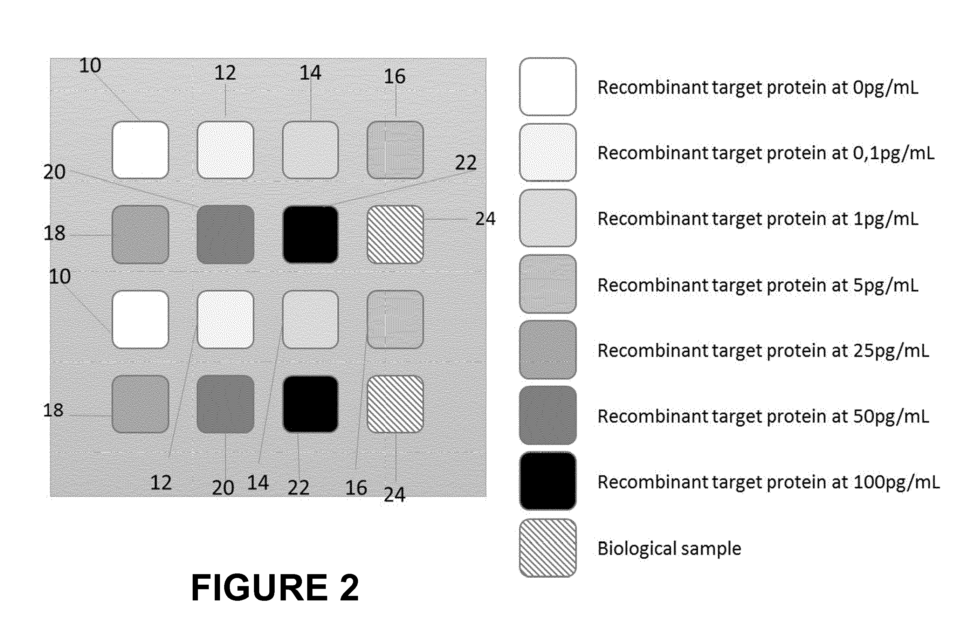

A picture of the recombinant target proteins in the wells and their quantity is shown in Figure 2.
The wells were washed with 1X PBS and 0.1%Tween. The wells were then hybridized for 2H with a mix composed of the following detection molecules

-   Anti IL1 alpha 2μL

-   Anti-IL6 2μL
    Anti-IL-10 2μL
    Anti-IL 17 2μL
    Anti-TNF alpha 2μL

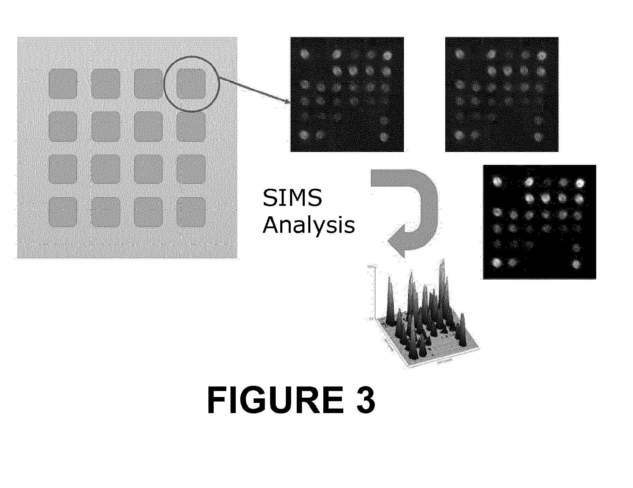

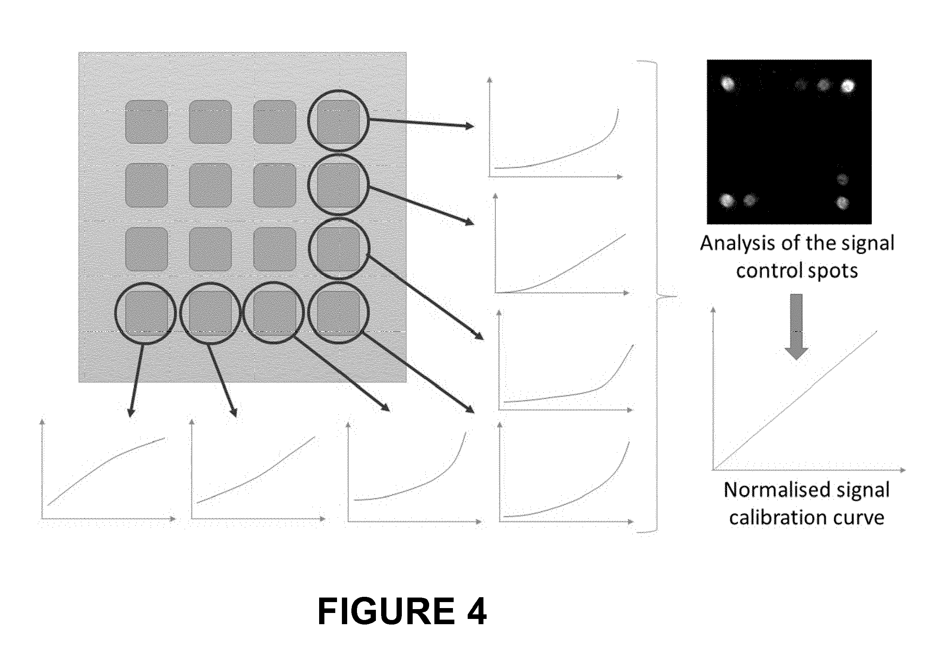

The silicon wafer (all of the wells) was washed with 1X PBS and 0.1% Tween, followed by washing with PBS 1X and then washed with HPLC grade distilled H$_2$O.
The silicon wafer was dried under air flow and stored under vacuum at 4°C
SIMS analysis was carried out as follows. The silicon wafer was installed in the SIMS analyser, put under high vacuum and all the wells position were programmed to be analysed.
For each well, the entire surface was analysed by D-SIMS. The detector was tuned on the mass 126.9 (Iodine label).This resulted in a matrix of 300 columns (number of pixels in width) and 300 lines (number of pixels in height). For each position (x,y) the value corresponded to the quantity of the secondary ions generated by the ionisation of the iodine and detected by the analyser.
Measurements of the signal on each spot of the well is shown in Figure 3.
An intra well signal control curve was obtained by extracting the value of the spots signal control molecule to determine a signal control curve (Quantity of label on the x-axis / Signal on the y-axis).
An inter well comparison was then performed using the signal control curves, which were correlated with each other and normalized as shown in Figure 4. All the different wells were compared with each other independently of the SIMS performance during the analysis.
All the data were normalised and formulated in an excel Table 1 as shown below:

**Table 1**

| Sample | Conc | Anti-IL1 | | | | | Anti-IL6 | | | | | | |
|---|---|---|---|---|---|---|---|---|---|---|---|---|---|
| | | Spot 01 | Spot 02 | Spot 03 | Spot 04 | Spot 05 | Spot 11 | Spot 12 | Spot 13 | Spot 14 | Spot 15 | Spot 21 | Spot 22 |
| BSA + 0 pg/ml Target proteins | 0 | 8 | 13 | 3 | 2 | 2 | 7 | 2 | 2 | 3 | 1 | 9 | 11 |
| BSA + 0,1 pg/ml Target proteins | 0,1 | 14 | 22 | 18 | 15 | 20 | 19 | 21 | 19 | 12 | 15 | 13 | 18 |
| BSA + 1 pg/ml Target proteins | 1 | 126 | 147 | 136 | 140 | 113 | 105 | 106 | 101 | 104 | 110 | 131 | 122 |
| BSA + 5 pg/ml Target proteins | 5 | 656 | 608 | 567 | 571 | 654 | 487 | 453 | 548 | 517 | 508 | 534 | 455 |
| BSA + 25 pg/ml Target proteins | 25 | 3157 | 3061 | 3334 | 2883 | 3131 | 2302 | 2514 | 2427 | 2738 | 2729 | 2930 | 2735 |
| BSA + 50 pg/ml Target proteins | 50 | 6090 | 6683 | 5686 | 6147 | 5718 | 4762 | 5398 | 4948 | 4842 | 4986 | 5372 | 4512 |
| BSA + 100 pg/ml Target proteins | 100 | 11402 | 12860 | 12356 | 12511 | 12307 | 10236 | 10801 | 10569 | 10484 | 10432 | 10429 | 9568 |
| Biological Sample | | 1213 | 1108 | 1297 | 1336 | 1146 | 2257 | 2737 | 2288 | 2515 | 2352 | 534 | 478 |
| BSA + 0 pg/ml Target proteins | 0 | 6 | 3 | 2 | 11 | 10 | 2 | 8 | 5 | 2 | 9 | 3 | 2 |
| BSA + 0,1 pg/ml Target proteins | 0,1 | 24 | 16 | 25 | 26 | 23 | 14 | 21 | 20 | 11 | 17 | 16 | 25 |
| BSA + 1 pg/ml Target proteins | 1 | 122 | 133 | 122 | 117 | 123 | 116 | 96 | 113 | 97 | 108 | 110 | 106 |
| BSA + 5 pg/ml Target proteins | 5 | 647 | 673 | 622 | 636 | 653 | 546 | 519 | 554 | 537 | 554 | 506 | 460 |
| BSA + 25 pg/ml Target proteins | 25 | 3150 | 3257 | 3311 | 3352 | 3372 | 2373 | 2324 | 2664 | 2332 | 2350 | 2438 | 2821 |
| BSA + 50 pg/ml Target proteins | 50 | 6220 | 6547 | 6491 | 6207 | 5530 | 5450 | 4543 | 4990 | 4970 | 5065 | 6152 | 5657 |
| BSA + 100 pg/ml Target proteins | 100 | 12104 | 12594 | 13209 | 11616 | 11271 | 10678 | 9518 | 10353 | 9512 | 9099 | 11405 | 10786 |
| Biological Sample | | 1176 | 1223 | 1299 | 1181 | 1119 | 2758 | 2376 | 2277 | 2332 | 2423 | 601 | 455 |

| Sample | Anti-IL10 | | | Anti-IL17 | | | | | Anti-TNF | | | | |
|---|---|---|---|---|---|---|---|---|---|---|---|---|---|
| | Spot 23 | Spot 24 | Spot 25 | Spot 31 | Spot 32 | Spot 33 | Spot 34 | Spot 35 | Spot 41 | Spot 42 | Spot 43 | Spot 44 | Spot 45 |
| BSA + 0 pg/ml Target proteins | 12 | 6 | 12 | 4 | 9 | 5 | 2 | 3 | 3 | 1 | 1 | 9 | 2 |
| BSA + 0,1 pg/ml Target proteins | 21 | 18 | 10 | 18 | 14 | 12 | 10 | 9 | 13 | 16 | 13 | 18 | 8 |
| BSA + 1 pg/ml Target proteins | 129 | 108 | 99 | 78 | 94 | 99 | 69 | 78 | 76 | 80 | 98 | 67 | 74 |
| BSA + 5 pg/ml Target proteins | 508 | 634 | 610 | 384 | 429 | 432 | 314 | 378 | 321 | 447 | 444 | 331 | 442 |
| BSA + 25 pg/ml Target proteins | 2803 | 2859 | 2878 | 1987 | 1639 | 1540 | 1852 | 1718 | 1741 | 2126 | 1577 | 2051 | 2003 |
| BSA + 50 pg/ml Target proteins | 5960 | 6083 | 5586 | 3216 | 3819 | 3646 | 4114 | 3617 | 4142 | 4145 | 4270 | 4436 | 4043 |
| BSA + 100 pg/ml Target proteins | 12336 | 9553 | 12489 | 6596 | 8434 | 8137 | 6882 | 8234 | 6791 | 7025 | 8529 | 7505 | 7829 |
| Biological Sample | 555 | 530 | 496 | 372 | 450 | 399 | 315 | 324 | 328 | 264 | 277 | 327 | 256 |
| BSA + 0 pg/ml Target proteins | 11 | 8 | 10 | 1 | 2 | 1 | 6 | 2 | 8 | 9 | 1 | 4 | 5 |
| BSA + 0,1 pg/ml Target proteins | 17 | 23 | 15 | 11 | 7 | 13 | 18 | 17 | 11 | 7 | 13 | 14 | 13 |
| BSA + 1 pg/ml Target proteins | 104 | 109 | 108 | 78 | 96 | 69 | 78 | 71 | 68 | 71 | 96 | 82 | 89 |
| BSA + 5 pg/ml Target proteins | 558 | 531 | 584 | 330 | 344 | 304 | 404 | 304 | 387 | 358 | 405 | 420 | 424 |
| BSA + 25 pg/ml Target proteins | 2470 | 2867 | 2552 | 1906 | 1849 | 1867 | 2243 | 2043 | 1823 | 1887 | 1970 | 1538 | 2084 |
| BSA + 50 pg/ml Target proteins | 5878 | 5493 | 5489 | 4066 | 3415 | 3802 | 3281 | 3466 | 4028 | 4366 | 3531 | 3912 | 3594 |
| BSA + 100 pg/ml Target proteins | 11516 | 9600 | 10904 | 7167 | 8424 | 7269 | 6495 | 8049 | 8940 | 7994 | 6448 | 8099 | 8435 |
| Biological Sample | 585 | 635 | 637 | 446 | 415 | 390 | 412 | 354 | 367 | 379 | 259 | 333 | 382 |

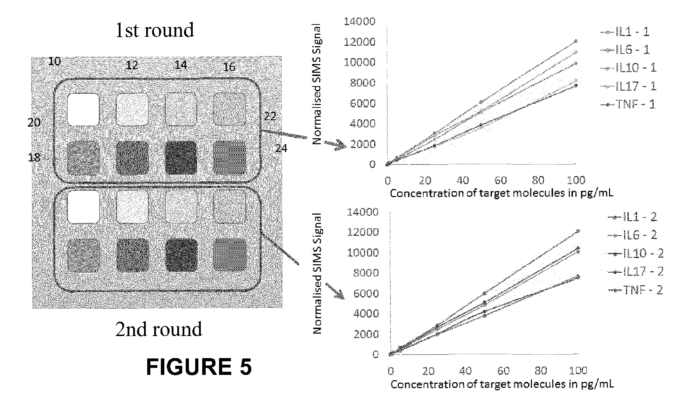

A calibration curve of the target molecules was then formulated using the different calibration samples to establish a calibration curve of the different target molecules (x-axis quantity of target molecules and y-axis normalised SIMS signal) as shown in Figure 5.

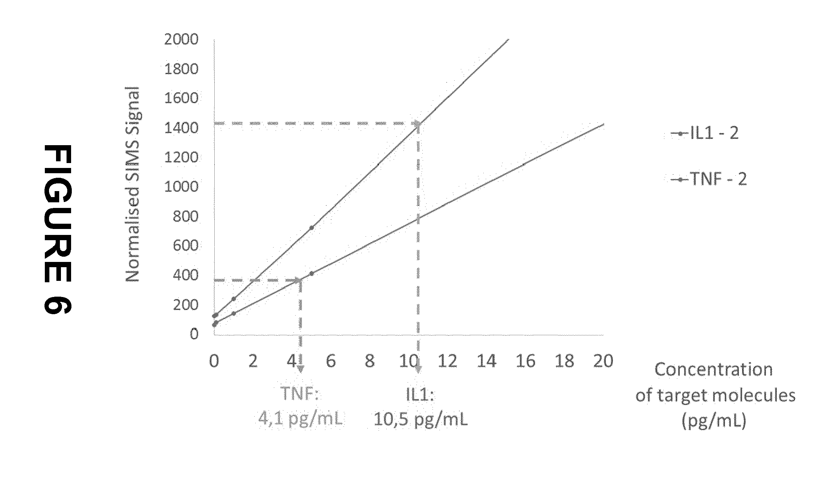

The signal of the different target molecules of the biological sample were plotted on the curve, the concentration value resulting in that corresponding to the concentration in the sample as shown in Figure 6.

**Example 3: Realization of a Biochip containing Iodine-label Signal Control Molecules and Bromine-label Capture Molecules**

A. Materials: The materials that were used to fabricate the biochip were the following:

(1) Silicon wafers (18 mm x 18 mm) (Siltronix) functionalized with epoxysilane coating (Sigma). There are 16 different zones (4x4) on the wafer with a size of 4 mm x4 mm. These zones are called wells.
(2) Capture molecules: antibodies for the following target molecules labelled with a click chemistry couple NHS-PEG-Azide/Alkyne, 2-bromo-benzene:

a. Anti IL1 alpha (R&D Systems MAB200)
b. Anti-IL6 (R&D Systems MAB206)
c. Anti-IL-10 (R&D Systems MAB2172)
d. Anti-IL 17 (R&D Systems MAB1248)
e. Anti-TNF (R&D Systems MAB610)

(3) Signal control molecule : An antibody labelled with a click chemistry coupled NHS-PEG-Azide/Alkyne, 2-iodo-benzene

a. Non-specific control (AbD Serotec HCA096): a non relevant antibody

(4) Dilution molecule: an antibody labelled with a click chemistry coupled NHS-PEG-Azide/Alkyne, 2-bromo-benzene

a. Non-specific control (AbD Serotec HCA096): a non relevant antibody

(5) Protein Printing Buffer 2X, Nexterion®
(6) BSA fraction V (Sigma Aldrich)
(7) PBS buffer

B. The biochips were formulated in the following manner:
In 384 well plate, the different antibodies were diluted in protein printing buffer 2X as follows:

o Well A1 : 10μL of antibody anti IL1 at 1 mg/mL + 10μL of protein printing buffer 2X
o Well A2 : 10μL of antibody anti IL6 at 1 mg/mL + 10μL of protein printing buffer 2X
o Well A3 : 10μL of antibody anti IL10 at 1 mg/mL + 10μL of protein printing buffer 2X
o Well A4 : 10μL of antibody anti IL17 at 1 mg/mL + 10μL of protein printing buffer 2X
o Well A5 : 10μL of antibody anti TNF at 1mg/mL + 10μL of protein printing buffer 2X
o Well A6 : 10μL of labelled non-specific control at 1 mg/mL + 10μL of protein printing buffer 2X
o Well A7 : 5μL of non-specific control at 1 mg/mL + 5μL of labelled non-specific control at 1 mg/mL + 10μL of protein printing buffer 2X
o Well A8 : 7.5μL of non-specific control at 1 mg/mL + 2.5μL of labelled non-specific control at 1 mg/mL + 10μL of protein printing buffer 2X
o Well A9 : 10μL of antibody non-specific control at 1 mg/mL + 10μL of protein printing buffer 2X

The well plate and the silicon wafers were placed in a SciFLEXArrayer (Scienion). The arrayer, after being programmed appropriately, fabricated the arrays following Figure 1.
The printed arrays were stored at 4°C overnight and then stored under vacuum at room temperature.

**Example 4: Test of the Quantification of the Target Molecules in a Biological Sample-Double label Normalisation**

A. Materials: The materials used in this example were the following:

(1) One Array fabricated in Example 3.
(2) Recombinant Molecules: The target molecules used for calibration curve were the following:

a. IL1 alpha (R&D Systems 200-LA-002)
b. IL6 (R&D Systems 206-IL-010)
c. IL-10 (R&D Systems 217-IL-005)
d. IL 17 (R&D Systems 317-ILB-005)
e. TNF alpha (R&D Systems 210-TA-005)

(3) Detection molecules: The detection molecules used were antibodies for the same target molecules coupled with a click chemistry coupled NHS-PEG-Azide/Alkyne, 2-iodo-benzene:

a. Anti IL1 alpha (R&D Systems AF-200-NA)
b. Anti-IL6 (R&D Systems AF-206-NA)
c. Anti-IL-10 (R&D Systems AF-217-NA)
d. Anti-IL 17 (R&D Systems AF-317-NA)
e. Anti-TNF alpha (R&D Systems AF-210-NA)

(4) BSA fraction V (Sigma Aldrich)
(5) PBS buffer

The hybridization was carried out as follows. The wells were first blocked with a solution containing 1% BSA in a 1X PBS buffer. The different wells of the silicon wafer were incubated for 2H with different samples containing various quantities of target molecules as set forth in Figure 2. These molecules will interact with the capture molecules.

a. Recombinant Target molecules (IL1, IL6, IL10 IL17 & TNF) at 0 pg/mL (10)
b. Recombinant Target molecules (IL1, IL6, IL10 IL17 & TNF) at 0.1 pg/mL (12)
c. Recombinant Target molecules (IL1, IL6, IL10 IL17 & TNF) at 1 pg/mL (14)
d. Recombinant Target molecules (IL1, IL6, IL10 IL17 & TNF) at 5 pg/mL (16)
e. Recombinant Target molecules (IL1, IL6, IL10 IL17 & TNF) at 25 pg/mL (18)
f. Recombinant Target molecules (IL1, IL6, IL10 IL17 & TNF) at 50 pg/mL (20)
g. Recombinant Target molecules (IL1, IL6, IL10 IL17 & TNF) at 100 pg/mL (22)
h. Reference 24 is the biological sample.

All of the wells were washed with 1X PBS and 0.1 % Tween. The wells were hybridized for 2H with a mix of the following detection molecules :

- Anti IL1 alpha 2$\mu$L
- Anti-IL6 2$\mu$L
- Anti-IL-10 2$\mu$L
- Anti-IL 17 2$\mu$L
- Anti-TNF alpha 2$\mu$L

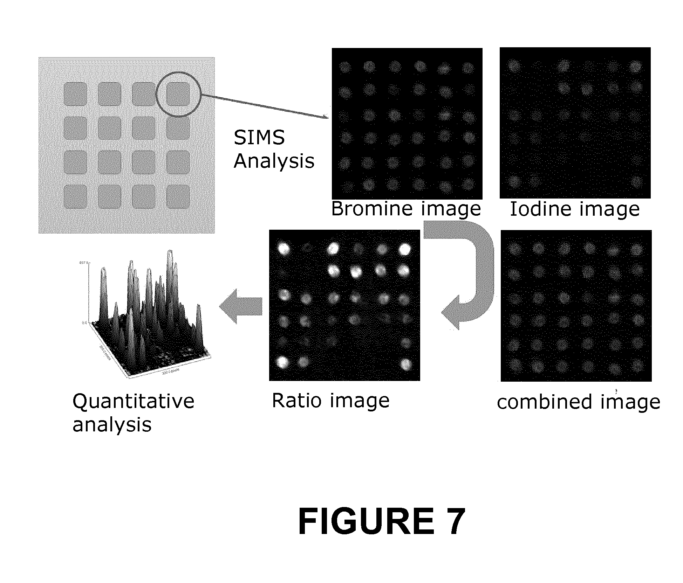

The silicon wafer (all of the wells) was washed with 1 X PBS and 0.1% Tween, followed by washing with PBS 1X and then washed with HPLC grade distilled $H_2O$.
The silicon wafer was dried under air flow and stored under vacuum at 4°C.
SIMS analysis was carried out as follows. The biochip was installed in the SIMS analyser, put under high vacuum and all the wells position were programmed to be analysed.
For each well, all the surface was analysed by D-SIMS. The detector was tuned on the mass 126.9 (Iodine label) and the mass 81 (Bromine Label).This resulted in a matrix of 300 columns (number of pixels in width) and 300 lines (number of pixels in height). For each position (x,y) the value corresponds to the quantity of the specific secondary ion detected by the analyser.
Background correction of the matrix was performed by a ratio procedure between iodine image and bromine image as set forth in Figure 7. Iodine and bromine images are represented in the color grey. However, if color could have been used, spots on bromine image should appear in red level and iodine image should appear in green level. Then, the combined image should then appear with a combination of red and green levels.

Measurements of the signal on each spot of the well was undertaken. An intra well signal control curve was obtained by extracting the value of the spots signal control molecule to determine a signal control curve (Quantity of label on the x-axis / Signal on the y-axis).

An inter well comparison was then made using the signal control curves, which were correlated with each other and normalized. All the different wells were compared with each other independently of the SIMS performance during the analysis.

All the data were normalized and formulated in an excel table similar to Table 1.

A calibration curve of the target molecules was then formulated using the different calibration samples to establish a calibration curve of the different target molecules (x-axis quantity of target molecules and y-axis normalized SIMS signal).

The signal of the different target molecules of the biological sample were plotted on the curve, the concentration value resulting in that corresponding to the concentration in the sample.

While the invention has been described in terms of various preferred embodiments, the skilled artisan will appreciate that various modifications, substitutions, omissions and changes may be made without departing from the scope thereof. Accordingly, it is intended that the scope of the present invention be limited by the scope of the claims, including equivalents thereof.

**Claims**

1. A biochip for use in surface mass spectrometry or similar spectroscopic techniques for quantitating a target molecule in a test sample comprising :

   a substrate to which are attached at least one assay spot(s) containing capture molecules and comprising at least three control spots containing a signal control molecule each control spot having different concentrations of signal control molecules.

2. The biochip according to Claim 1, wherein said capture and signal control molecules are attached to said substrate through a functionalized moiety.

3. The biochip according to Claim 2, wherein said capture and signal control molecules are functionalized for attachment to said substrate.

4. The biochip according to any one of Claims 1 to 3, wherein the signal control molecule is labeled with a first signal source.

5. The biochip according to Claim 1, wherein said at least one assay spot containing said capture molecules binds to a target molecule in a test sample.

6. The biochip according to Claim 5, wherein said target molecule selected from the group of a protein, a peptide, a nucleic acid, a carbohydrates, a lipid, a polysaccharide, a glycoprotein, a hormone, an antigen, a pathogen, a toxic substance, a drug, a dye, a nutrient, an enzyme, a rheumatoid factor, a tumor marker, a microorganism, an opiate, a viral epitope, cholesterol, a heavy metal, a vitamin, an allergy, a neurodegenerative disorder, an electrolyte, a glycan, a polyamine, a fatty acid, a sugar, a chemical trace metals and mixtures thereof.

7. The biochip according to any one of Claims 1 to 6, wherein the size of the at least one spot range from 1 μm to 4.0 mm in diameter.

8. The biochip according to any one of Claims 1 to 7, wherein the spots on said biochip are measured using a surface mass spectrometer or using similar spectroscopic techniques.

9. A kit for use in surface mass spectrometry for quantitating test samples comprising:

   a) the biochip according to any one of Claims 1 to 8; and
   b) a detection molecule to which is attached a detection signal source.

10. A kit for use in surface mass spectrometry for quantitating test samples comprising:

a) the biochip according to any one of Claims 1 to 8; and
b) reagents to graft detection a detection signal source to detection or target molecules.

11. The kit according to Claim 9 or 10, wherein said detection signal source is an element of I, Br, F, S, Au, Fe, Se, As, P, B, Cu, Ag, Zn, Ni or a lanthanide.

12. The kit according to any one of Claims 9 to 11, wherein the signal source is one of a group of atoms having a relative sensitivity factor of <1E23 under negative ion bombardment, or a relative sensitivity factor of <1E24 under positive ion bombardment.

13. A biochip according to any one of Claims 1 to 8 for use in quantitating a target molecule in at least one assay spot.

14. A method of quantitating a target molecule in at least one assay sample comprising:

(f) producing the biochip according to any one of Claims 1 to 8;
(g) placing at least one target molecule on designated places on said biochip allowing the at least one target molecule to interact with the capture molecule;
(h) placing at least one detection molecule on designated places on the biochip;
(i) detecting the presence of the at least one detection molecule on said biochip by counting detecting molecules using a surface mass spectrometer; and
(j) calculating the quantity of said at least one target molecule on said biochip.

15. The method according to Claim 14, wherein said surface mass spectrometer or similar spectroscopic techniques are selected from the group of nano-Secondary Ion Mass Spectrometer (nano-SIMS), Time of Flight Secondary Ion Mass Spectrometer (TOF-SIMS), Time of Flight Matrix-Assisted Laser Desorption/Ionization (Maldi-Tof), electron spray ionization mass spectrometry (ESIMS), Ion Mobility Spectrometer (IMS), Secondary Ion Mass Spectrometer (SIMS), Matrix-Assisted laser Desorption/Ionization (MALDI), Surface Enhanced Laser Desorption/Ionization (SELDI), Surface-Assisted Laser Desorption/Ionization (SALDI), Auger Electron Spectroscopy (AES), Energy-Dispersive X-ray Spectroscopy (EDS or EDX), X-ray Photoelectron Spectroscopy (XPS) and Dynamic Secondary Ion Mass Spectrometer (D-SIMS).

FIGURE 1

**FIGURE 2**

SIMS
Analysis

# FIGURE 3

Analysis of the signal control spots

Normalised signal calibration curve

**FIGURE 4**

FIGURE 5

FIGURE 6

SIMS
Analysis

Bromine image    Iodine image

Quantitative
analysis    Ratio image    combined image

# FIGURE 7

Europäisches
Patentamt

European
Patent Office

Office européen
des brevets

# EUROPEAN SEARCH REPORT

Application Number

EP 14 30 6835

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 03/031976 A2 (RANDOX LAB LTD [GB]; LAMONT JOHN VICTOR [GB]; MCCONNELL ROBERT IVAN [G) 17 April 2003 (2003-04-17) * example 1 * | 1-10,13, 14 | INV. G01N33/68 G01N33/543 |
| X | WO 01/83825 A2 (BLOOD RES CENTER [US]) 8 November 2001 (2001-11-08) * claims 33-38 * | 1-10,13, 14 | |
| X | US 2003/153013 A1 (HUANG RUO-PAN [US]) 14 August 2003 (2003-08-14) * paragraphs [0005], [0022]; figure 11 * | 1-15 | |

TECHNICAL FIELDS
SEARCHED (IPC)

G01N

The present search report has been drawn up for all claims

| Place of search | Date of completion of the search | Examiner |
|---|---|---|
| The Hague | 29 April 2015 | Gundlach, Björn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 ...............................................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 14 30 6835

This annex lists the patent family members relating to the patent documents cited in the above-mentioned European search report.
The members are as contained in the European Patent Office EDP file on
The European Patent Office is in no way liable for these particulars which are merely given for the purpose of information.

29-04-2015

| Patent document cited in search report | | | Publication date | Patent family member(s) | | | Publication date |
|---|---|---|---|---|---|---|---|
| WO 03031976 | A2 | | 17-04-2003 | CN | 1589404 | A | 02-03-2005 |
| | | | | EP | 1434995 | A2 | 07-07-2004 |
| | | | | JP | 4920173 | B2 | 18-04-2012 |
| | | | | JP | 2005504988 | A | 17-02-2005 |
| | | | | US | 2004241700 | A1 | 02-12-2004 |
| | | | | WO | 03031976 | A2 | 17-04-2003 |
| WO 0183825 | A2 | | 08-11-2001 | AT | 418733 | T | 15-01-2009 |
| | | | | AU | 5945501 | A | 12-11-2001 |
| | | | | CA | 2408094 | A1 | 08-11-2001 |
| | | | | EP | 1307743 | A2 | 07-05-2003 |
| | | | | JP | 4222756 | B2 | 12-02-2009 |
| | | | | JP | 2003532091 | A | 28-10-2003 |
| | | | | US | 2002015958 | A1 | 07-02-2002 |
| | | | | US | 2005142666 | A1 | 30-06-2005 |
| | | | | US | 2008139405 | A1 | 12-06-2008 |
| | | | | WO | 0183825 | A2 | 08-11-2001 |
| US 2003153013 | A1 | | 14-08-2003 | US | 2003153013 | A1 | 14-08-2003 |
| | | | | US | 2007054326 | A1 | 08-03-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010009387 A
- US 6040193 A
- US 5885837 A

**Non-patent literature cited in the description**

- **OLLE et al.** *Mol And Cell Prot,* 2005, vol. 4, 11 1664-1672
- **YAM et al.** *J. Colloid. Interf Sci,* 2006, vol. 296, 118-130
- **HODNELAND et al.** *PNAS,* 2002, vol. 99 (8), 5048-5052
- **CAMARERO et al.** *J. Am Chem Soc,* 2004, vol. 126 (45), 14730-1
- **SUN et al.** *Bioconjugate Chem,* 2006, vol. 17, 52-57
- **KWON et al.** *Angew Chem Int Ed,* 2006, vol. 45 (11), 1726-9
- **KOLODYCH et al.** *Angewandte Chemie International Edition,* November 2013, vol. 52 (46), 12056-12060